# NEUE EUROPÄISCHE PATENTSCHRIFT

(11) **EP 2 448 996 B2**
(45) Veröffentlichungstag und Bekanntmachung des Hinweises auf die Entscheidung über den Einspruch: **12.04.2017**
(45) Hinweis auf die Patenterteilung: 28.08.2013
(21) Anmeldenummer: 10729815.0
(22) Anmeldetag: 30.06.2010
(51) Int. Cl.: C08G 65/26, C08G 65/332, C08G 18/48

(54) **VERFAHREN ZUR HERSTELLUNG VON POLYETHERPOLYOLEN MIT PRIMÄREN HYDROXYL-ENDGRUPPEN**
METHOD FOR THE PRODUCTION OF POLYETHER POLYOLS COMPRISING TERMINAL PRIMARY HYDROXYL GROUPS
PROCÉDÉ DE PRÉPARATION DE POLYÉTHERPOLYOLS COMPORTANT DES GROUPES TERMINAUX HYDROXYLE

(30) Priorität: 03.07.2009 DE 102009031584
(43) Veröffentlichungstag der Anmeldung: 09.05.2012
(73) Patentinhaber: Covestro Deutschland AG, 51373 Leverkusen (DE)
(72) Erfinder: NEFZGER, Hartmut, 50259 Pulheim (DE); BAUER, Erika, 41363 Jüchen (DE); HOFMANN, Jörg, 47800 Krefeld (DE); LORENZ, Klaus, 41539 Dormagen (DE); HAHN, Norbert, 50226 Frechen (DE)
(74) Vertreter: Levpat
(86) Internationale Anmeldenummer: PCT/EP2010/003958
(87) Internationale Veröffentlichungsnummer: WO 2011/000560

(56) Entgegenhaltungen:
- EP-A1- 0 248 561
- WO-A2-2006/060273
- US-A- 4 487 853
- US-A- 4 582 926
- US-A- 6 066 683
- US-B1- 7 226 988
- US-B2- 7 186 867
- US-B2- 7 189 799
- O'CONNOR J.M. ET AL: 'New DMC Catalysts for Manufacturing Polyols' POLYURETHANES EXPO 2001 2001, Seite 227
- IONESCU M.: 'Chemistry and Technology of Polyols for Polyurethanes', 2005, RAPRA TECHNOLOGY LIMITED Seite 173
- WEGENER G. ET AL: 'Trends in industrial catalysis in the polyurethane industry' APPLIED CATALYSIS Bd. 221, 2001, Seiten 303 - 335

## Beschreibung

Die vorliegende Erfindung betrifft ein Verfahren zur Herstellung von Polyetherpolyolen mit primären Hydroxyl-Endgruppen, umfassend die Schritte der Reaktion einer aktive Wasserstoffatome aufweisenden Starterverbindung mit einem Epoxid unter Doppelmetallcyanid-Katalyse, der Reaktion des erhaltenen Produktes mit einem zyklischen Carbonsäureanhydrid sowie der Reaktion dieses erhaltenen Produktes mit Ethylenoxid in Gegenwart eines Katalysators wie in Anspruch 1 definiert, welcher pro Molekül mindestens ein Stickstoffatom umfasst, wobei nicht-zyklische, identisch substituierte tertiäre Amine ausgenommen sind. Die Erfindung betrifft weiterhin gemäß diesem Verfahren erhältliche Polyetherpolyole, Zusammensetzungen umfassend diese Polyole sowie Polyurethanpolymere auf der Basis dieser Polyole.

Durch Doppelmetallcyanid-Katalyse (DMC-Katalyse) hergestellte langkettige Polyetherpolyole werden auch als IMPACT-Polyether bezeichnet. Sie weisen systembedingt überwiegend sekundäre Hydroxylendgruppen auf. Der Einsatz von Ethylen-/Propylenoxidgemischen (EO/PO) ist nur bis zu einem bestimmten Anteil an EO möglich; somit sind langkettige Polyetherpolyole mit überwiegend primären Hydroxylendgruppen nach dem Impactverfahren nicht zugänglich. Stattdessen werden derartige Polyether entweder dadurch erhalten, dass ausschließlich mit konventioneller Basenkatalyse (zum Beispiel KOH) katalysiert wird oder in zweistufiger Verfahrensweise auf einen mittels DMC-Katalyse erhaltenen IMPACT-PO-Polyether, gegebenenfalls einen PO/EO-Mischpolyether beziehungsweise einen PO/EO-Mischendblock aufweisenden Polyether, unter KOH-Katalyse ein EO-Endblock aufpolymerisiert wird.

Das KOH-Verfahren weist generell den Nachteil auf, dass dieser Katalysator aufwendig, zum Beispiel durch Neutralisation und Filtration, abgetrennt werden muss. Darüber hinaus entstehen insbesondere bei langkettigen Polyethern unerwünschte olefinische Endgruppen als Nebenprodukte. Derartige olefinische Endgruppen oder Allylether-Endgruppen vermindern die Funktionalität dieser Polyether und erschweren ihren Einsatz in bestimmten Anwendungen. Auch führen sie zu Polyurethan- (PUR-)Produkten, die qualitativ schlechter sind.

US 4,487,853 offenbart ein Verfahren zur Herstellung eines Polyetheresterpolyols mit einem hohen Gehalt an primären Hydroxylgruppen. In diesem Verfahren werden bei einer Temperatur von 50 °C bis 125 °C a) das Reaktionsprodukt eines Kondensats eines Polyols mit einem Alkylenoxid mit einem zyklischen Carbonsäureanhydrid und b) Ethylenoxid zur Reaktion gebracht. Das Kondensat wird aus einem Polyol mit 2 bis 8 Hydroxylgruppen und einem Äquivalentgewicht von 30 bis 45 sowie einem Alkylenoxid mit 2 bis 4 Kohlenstoffatomen und Mischungen davon erhalten. Das Kondensat weist ein Äquivalentgewicht von 500 bis 10000 auf. Nach Reaktion mit dem zyklischen Carbonsäureanhydrid wird ein Halbester erhalten. Die Reaktion von a) mit Ethylenoxid findet in Gegenwart einer wirksamen Menge eines Amins, Oxids oder zweiwertigen Metallkatalysators statt. Das Verhältnis der Äquivalente des Anhydrids zu den Äquivalenten des Kondensats liegt im Bereich von ungefähr 1:1 bis ungefähr 1:2 und das Molverhältnis von Ethylenoxid zu Anhydrid liegt im Bereich von ungefähr 2:1 bis ungefähr 1,5:1. Offenbart wird weiterhin ein Polyurethan aus der Reaktion eines organischen Polyisocyanats mit solchen Polyolen.

Es wird jedoch in US 4,487,853 nicht beschrieben, wie unter DMC-Katalyse hergestellte Polyetherpolyole unter möglichst geringem Verfahrensaufwand in Polyole mit primären Hydroxylendgruppen überführt werden können. Folglich besteht weiterhin der Bedarf an alternativen Herstellungsverfahren für Polyetherpolyole mit primären Hydroxyl-Endgruppen und insbesondere an solchen Verfahren, welche mit DMC-Katalyse hergestellte Polyether umsetzen.

Gegenstand der Erfindung ist ein Verfahren zur Herstellung von Polyetherpolyolen mit primären Hydroxyl-Endgruppen, umfassend die Schritte:
1. Reaktion einer aktive Wasserstoffatome aufweisenden Starterverbindung mit einem Epoxid der allgemeinen Formel (1): 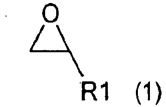 wobei R1 für Wasserstoff, einen Alkylrest oder einen Arylrest steht und mit der Maßgabe, dass ≥ 0 Gewichts-% bis ≤ 30 Gewichts-%, bezogen auf die Gesamtmenge des eingesetzten Epoxids (1), Ethylenoxid sind,
   wobei die Reaktion in Gegenwart eines Doppelmetallcyanid-Katalysators durchgeführt wird und wobei das Rohprodukt dieser Reaktion mit Ausnahme eines möglichen Destillationsschrittes keine weitere Aufreinigung erfährt;
2. Reaktion des in Schritt 1. erhaltenen Produkts mit einem zyklischen Carbonsäureanhydrid; und
3. Reaktion des in Schritt 2. erhaltenen Produkts mit Ethylenoxid in Gegenwart eines Katalysators, wie in Anspruch 1 definiert, welcher pro Molekül mindestens ein Stickstoffatom umfasst, wobei nicht-zyklische, identisch substituierte tertiäre Amine ausgenommen sind.

Sofern im Rahmen der vorliegenden Erfindung von als Endprodukt herzustellenden Polyetherpolyolen die Rede ist, schließt der Begriff selbstverständlich solche Polyetherpolyole ein, welche als Folge des erfindungsgemäßen Verfahrens auch Estereinheiten umfassen.

Ein Vorteil des erfindungsgemäßen Verfahrens ist, dass unter DMC-Katalyse hergestellte Polyether, welche auch bei hohen mittleren Molekülmassen keine oder eine technisch unbedeutende Abweichung der tatsächlichen von der idealen OH-Funktionalität zeigen, zu Polyolen mit einem höheren Anteil an primären OH-Gruppen reagieren. Dadurch, dass nach dem ersten Schritt die Entfernung des Katalysators entfällt, lässt sich eine Vereinfachung des Gesamtverfahrens erreichen.

Als aktive Wasserstoffatome aufweisende Starterverbindungen im Schritt 1. werden vorzugsweise Verbindungen mit (zahlenmittleren) Molekulargewichten von ≥ 18 g/mol bis ≤ 2000 g/mol und ≥ 1 bis ≤ 8 Hydroxylgruppen eingesetzt. Beispiele hierfür sind Ethylenglykol, Diethylenglykol, Triethylenglykol, 1,2-Propylenglykol, Dipropylenglykol, 1,4-Butandiol, Hexamethylenglykol, Bisphenol A, Bisphenol F, Trimethylolpropan, Glycerin, Ricinusöl, Pentaerythrit, Sorbit, Rohrzucker, abgebaute Stärke und/oder Wasser.

Es können weiterhin auch solche aktive Wasserstoffatome aufweisenden Starterverbindungen eingesetzt werden, die zum Beispiel durch konventionelle Alkalikatalyse aus den zuvor genannten niedermolekularen Starterverbindungen hergestellt wurden und oligomere Alkoxylierungsprodukte mit (zahlenmittleren) Molekulargewichten von ≥ 200 g/mol bis ≤ 2000 g/mol darstellen.

Das Epoxid der allgemeinen Formel (1) ist ein terminales Epoxid mit einem Substituenten R1, welcher Wasserstoff, ein Alkylrest oder ein Arylrest sein kann. Der Begriff "Alkyl" umfasst allgemein im Zusammenhang der gesamten Erfindung Substituenten aus der Gruppe n-Alkyl wie Methyl, Ethyl oder Propyl, verzweigtes Alkyl und/oder Cycloalkyl. Der Begriff "Aryl" umfasst allgemein im Zusammenhang der gesamten Erfindung Substituenten aus der Gruppe einkernige Carbo- oder Heteroarylsubstituenten wie Phenyl und/oder mehrkernige Carbo- oder Heteroarylsubstituenten. Es ist möglich, dass auch Mischungen verschiedener Epoxide im erfindungsgemäßen Verfahren eingesetzt werden können, solange die Bestandteile der Epoxidmischung alle unter die allgemeine Formel (1) fallen. Bei Verwendung von Mischungen verschiedener Epoxide ist es auch möglich, das Mischungsverhältnis der Epoxide während der Dosierung stufenweise oder kontinuierlich zu verändern.

Die für Schritt 1. des erfindungsgemäßen Verfahrens geeigneten Doppelmetallcyanid-Katalysatoren haben vorzugsweise die allgemeine Formel M¹ₐ[M²(CN)_{b}(A)_{c}]_{d} · fM¹_{g}X_{z} · h (H₂O) · e L.

Hierbei ist M¹ ein Metallion ausgewählt aus der Gruppe enthaltend Zn²⁺, Fe²⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺ und/oder Cd²⁺. M² steht für ein Metallion ausgewählt aus der Gruppe enthaltend Fe²⁺, Fe³⁺, Co²⁺, Co³⁺, Mn²⁺, Mn₃₊, V⁴⁺, V⁵⁺, Cr²⁺, Cr³⁺, Rh³⁺, Ru²⁺ und/oder Ir³⁺. M¹ und M² sind gleich oder verschieden.

A ist ein Anion ausgewählt aus der Gruppe enthaltend Halogenid, Hydroxid, Sulfat, Carbonat, Cyanid, Thiocyanat, Isocyanat, Cyanat, Carboxylat, Oxalat und/oder Nitrat. X ist ein Anion ausgewählt aus der Gruppe enthaltend Halogenid, Hydroxid, Sulfat, Carbonat, Cyanid, Thiocyanat, Isocyanat, Cyanat, Carboxylat, Oxalat und/oder Nitrat. L ist ein mit Wasser mischbarer Ligand ausgewählt aus der Gruppe enthaltend Alkohole Aldehyde, Ketone, Ether, Polyether, Ester, Harnstoffe, Amide, Nitrile und/oder Sulfide.

Die Zählvariablen a, b, c, d, g und z sind so ausgewählt, dass die Elektroneutralität der Verbindung gewährleistet ist. Weiterhin bedeuten e die Koordinationszahl des Liganden, f eine gebrochene oder ganze Zahl größer oder gleich 0 und h eine gebrochene oder ganze Zahl größer oder gleich 0.

Die für Schritt 1. des erfindungsgemäßen Verfahrens geeigneten DMC-Katalysatoren sind im Prinzip aus dem Stand der Technik bekannt (US 3,404109, US 3,829,505, US 3,941,849 und US 5,158,922). Bevorzugt eingesetzt werden verbesserte, hochaktive DMC-Katalysatoren, die zum Beispiel in US 5,470,813, EP 0 700 949 A2, EP 0 743 093 A1, EP 0 761 708 A2, WO 97/40086 A1, WO 98/16310 A1 und WO 00/47649 A1 beschrieben sind. Diese besitzen eine außerordentlich hohe Aktivität und ermöglichen die Herstellung von Polyetherpolyolen bei sehr geringen Katalysatorkonzentrationen. Ein typisches Beispiel sind die in EP 0 700 949 A2 beschriebenen hochaktiven DMC-Katalysatoren, die neben einer Doppelmetallcyanid-Verbindung wie Zinkhexacyanocobaltat(III) und einem organischen Komplexliganden wie tert.-Butanol noch einen Polyether mit einem zahlenmittlerem Molekulargewicht von größer als 500 g/mol enthalten.

Der DMC-Katalysator in Schritt 1. wird bevorzugt entsprechend der Lehre aus EP 0 700 949 A2 erhalten, auf die in vollem Umfang Bezug genommen wird. Der Katalysator kann als Komponenten eine Doppelmetallcyanid-Verbindung, also ein Reaktionsprodukt eines wasserlöslichen Metallsalzes und eines wasserlöslichen Metallcyanidsalzes, weiterhin einen organischen Komplexbildner L und ≥ 5 Gewichts-% bis ≤ 80 Gewichts-%, bezogen auf die Katalysatormenge, eines Polyethers mit einem Zahlenmittel der Molekülmasse von ≥ 500 g/mol umfassen.

Der Katalysator kann beispielsweise in einem Anteil, bezogen auf die Gesamtmasse von eingesetzter Starterverbindung und Epoxid (1), von ≥ 1 ppm bis ≤ 100 ppm und vorzugsweise von ≥ 10 ppm bis ≤ 50 ppm eingesetzt werden.

Die DMC-katalysierte Reaktion zwischen der Starterverbindung und dem Epoxid (1) in Schritt 1. erfolgt im allgemeinen bei Temperaturen von ≥ 20 °C bis ≤ 200° C, bevorzugt im Bereich von ≥ 40 °C bis ≤ 180 °C, besonders bevorzugt bei Temperaturen von ≥ 50 °C bis ≤ 150 °C. Die Reaktion kann bei Gesamtdrücken von 0,0001 bis 20 bar durchgeführt werden.

Die (zahlenmittleren) Molekulargewichte der im Schritt 1. des erfindungsgemäßen Verfahrens hergestellten Polyetherpolyole können ≥ 500 g/mol bis ≤ 100000 g/mol, bevorzugt ≥ 1000 g/mol bis ≤ 50000 g/mol, besonders bevorzugt ≥ 2000 g/mol bis ≤ 20000 g/mol betragen.

Die Reaktion im Schritt 1. kann kontinuierlich oder diskontinuierlich, zum Beispiel in einem Batch- oder im Semibatchverfahren, durchgeführt werden.

Im erfindungsgemäßen Verfahren ist vorgesehen, dass in Schritt 1. das Epoxid (1) zu höchstens 30 Gewichts-% Ethylenoxid enthält. Es wurde festgestellt, dass bei höheren Ethylenoxid-Gehalten keine zufriedenstellenden Reaktionsprodukte für die Weiterverarbeitung in den darauffolgenden Schritten des Verfahrens erhalten werden.

Im Rahmen der vorliegenden Erfindung ist vorgesehen, dass das Rohprodukt der Reaktion aus Schritt 1. mit Ausnahme eines möglichen Destillationsschrittes keine weitere Aufreinigung erfährt. Dieser Destillationsschritt ist folglich optional. Durch die Destillation kann beispielsweise nicht umgesetztes Epoxid (1) von dem erhaltenen Polyol entfernt werden. Aufreinigungsschritte, die auf das Produkt gerade nicht angewandt werden, wären zum Beispiel eine Filtration, eine Lösungsmittelextraktion oder eine chromatographische Reinigung. Hierin liegt ein Vorteil des erfindungsgemäßen Verfahrens, da kostenintensive Aufreinigungsschritte für gemäß dem KOH-Verfahren hergestellte Polyetherpolyole vermieden werden. Ein gesonderter Aufreinigungsschritt ist nicht nötig, da die Doppelmetallcyanid-Katalysatoren im Rohprodukt verbleiben können, ohne dass sie die Folgereaktionen stören und weiterhin in nur geringen Mengen benötigt werden.

In Schritt 2. des erfindungsgemäßen Verfahrens wird das, wenn überhaupt, nur destillativ gereinigte Produkt aus Schritt 1 weiter umgesetzt. Hierbei werden endständige Hydroxyl-Gruppen des erhaltenen Polyetherpolyols mit einem zyklischen Carbonsäureanhydrid reagiert. Man erhält unter Öffnung der Anhydridgruppe eine Esterbindung zu dem Polyetherpolyol sowie eine weitere freie Carboxylgruppe. Optional wird die Reaktion in Gegenwart eines Katalysators durchgeführt, welcher pro Molekül mindestens ein Stickstoffatom umfasst. Vorzugsweise ist dieses ein organisches Molekül, so dass es sich bei dem Katalysator um ein organisches Amin handelt. Ausgenommen sind jedoch nicht-zyklische, identisch substituierte tertiäre Amine. Ein Beispiel für solch ein nicht geeignetes Amin ist Triethylamin. Wenn ein Katalysator eingesetzt wird, ist er vorteilhafterweise derselbe Katalysator wie im nachfolgenden Schritt 3.

Die Menge des stickstoffhaltigen Katalysators, bezogen auf die Gesamtmasse des Reaktionsansatzes in Schritt 2., kann beispielsweise ≥ 10 ppm bis ≤ 10000 ppm, bevorzugt ≥ 50 ppm bis ≤ 5000 ppm und mehr bevorzugt ≥ 100 ppm bis ≤ 2000 ppm betragen. Die Reaktionstemperatur in Schritt 2. kann dabei ≥ 70 °C bis ≤ 150 °C und vorzugsweise ≥ 80 °C bis ≤ 135 °C betragen.

Schritt 3. des erfindungsgemäßen Verfahrens betrifft die Reaktion des in Schritt 2. erhaltenen Produkts mit Ethylenoxid. Durch die Reaktion der Carboxyl-Gruppen des Polyethers entstehen unter Ringöffnung Hydroxyalkylgruppen. Vorzugsweise reagieren ≥ 80%, ≥ 90% oder ≥ 95% der Carboxyl-Gruppen mit dem Epoxid und es wird vorzugsweise ein Anteil an primären Hydroxyl-Gruppen von ≥ 50 Mol-% bis ≤ 100 Mol-% oder von ≥ 60 Mol-% bis ≤ 85 Mol-% erhalten.

Erfindungsgemäß vorgesehen ist, dass diese Reaktion in Gegenwart eines Katalysators wie in Anspruch 1 definiert durchgeführt, welcher pro Molekül mindestens ein Stickstoffatom umfasst. Vorzugsweise ist dieses ein organisches Molekül, so dass es sich bei dem Katalysator um ein organisches Amin handelt. Erfindungsgemäß ausgenommen sind jedoch nicht-zyklische, identisch substituierte tertiäre Amine. Ein Beispiel für solch ein nicht geeignetes Amin ist Triethylamin.

Die Menge des stickstoffhaltigen Katalysators, bezogen auf die Gesamtmasse des Reaktionsansatzes in Schritt 3., kann beispielsweise ≥ 10 ppm bis ≤ 10000 ppm, bevorzugt ≥ 50 ppm bis ≤ 5000 ppm und mehr bevorzugt ≥ 100 ppm bis ≤ 2000 ppm betragen. Die Reaktionstemperatur in Schritt 3. kann dabei ≥ 70 °C bis ≤ 150 °C und vorzugsweise ≥ 80 °C bis ≤ 135 °C betragen.

Vorteilhafterweise schließt sich dieser Schritt unmittelbar an Schritt 2. an, so dass zu dem Reaktionsansatz aus Schritt 2. nach Ende der Reaktion mit dem zyklischen Carbonsäureanhydrid das Ethylenoxid hinzugefügt wird.

In einer Ausführungsform des erfindungsgemäßen Verfahrens ist die in Schritt 1. eingesetzte Starterverbindung ein Poly(oxyalkylen)-Polyol mit einer durchschnittlichen Funktionalität von ≥ 2,0 bis ≤ 5,0, einem Zahlenmittel der Molekülmasse von ≥ 62 g/mol bis ≤ 1000 g/mol und einer OH-Zahl von ≥ 100 mg KOH/g bis ≤ 1860 mg KOH/g. Die durchschnittliche Funktionalität kann auch ≥ 2,3 bis ≤ 4,0 betragen, das Zahlenmittel der Molekülmasse ≥ 100 g/mol bis ≤ 500 g/mol und die OH-Zahl 200 mg KOH/g bis ≤ 300 mg KOH/g. Die OH-Zahl lässt sich anhand der Norm DIN 53240 bestimmen.

In einer weiteren Ausführungsform des erfindungsgemäßen Verfahrens ist in dem Epoxid der allgemeinen Formel (1) R1 Wasserstoff, Methyl, Ethyl, n-Propyl, iso-Propyl, n-Butyl, sec-Butyl, iso-Butyl, tert-Butyl, Cyclohexyl und/oder Phenyl. Bevorzugt ist hierbei, dass R1 Methyl ist. Dann ist das eingesetzte Epoxid Propylenoxid. Ebenfalls bevorzugt sind Mischungen von Propylenoxid und Ethylenoxid, so dass gemischte Polyetherblöcke erhalten werden. Es können auch nacheinander mehrere Mischungen von Propylenoxid und Ethylenoxid mit unterschiedlichen Mischungsverhältnissen eingesetzt werden.

In einer weiteren Ausführungsform des erfindungsgemäßen Verfahrens umfasst der Doppelmetallcyanid-Katalysator in Schritt 1. Zink, Kobalt und tert.-Butanol. Vorzugsweise umfasst dieser Katalysator zusätzlich ≥ 5 Gewichts-% bis ≤ 80 Gewichts-%, bezogen auf die Katalysatormenge, eines Polyethers mit einem Zahlenmittel der Molekülmasse von ≥ 500 g/mol. Der Anteil des Polyethers kann auch ≥ 10 Gewichts-% bis ≤ 70 Gewichts-% und besonders bevorzugt ≥ 15 Gewichts-% bis ≤ 60 Gewichts-% betragen. Besonders geeignete Polyether sind beispielsweise Polyetherpolyole mit einer durchschnittlichen OH-Funktionalität von 2 bis 8 und einem Zahlenmittel der Molekülmasse von ≥ 1000 g/mol bis ≤ 10000 g/mol und vorzugsweise von ≥ 1000 g/mol bis ≤ 5000 g/mol. Als Beispiel genannt seien Poly(oxypropylen)polyole, insbesondere Diole und/oder Triole mit einem Zahlenmittel der Molekülmasse von ≥ 1000 g/mol bis ≤ 4000 g/mol.

In einer weiteren Ausführungsform des erfindungsgemäßen Verfahrens ist das in Schritt 2. eingesetzte zyklische Carbonsäureanhydrid ausgewählt aus der Gruppe umfassend Phthalsäureanhydrid, Tetrahydrophthalsäureanhydrid, Bernsteinsäureanhydrid und/oder Maleinsäureanhydrid.

Im erfindungsgemäßen Verfahren ist der in Schritt 3. eingesetzte Katalysator ausgewählt aus der Gruppe umfassend:
(A) Amine der allgemeinen Formel (2): 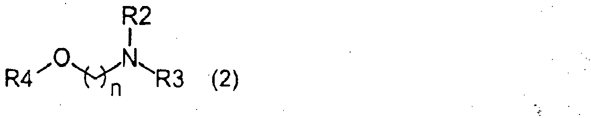 wobei gilt:
   R2 und R3 sind unabhängig voneinander Wasserstoff, Alkyl oder Aryl; oder
   R2 und R3 bilden gemeinsam mit dem sie tragenden N-Atom einen aliphatischen, ungesättigten oder aromatischen Heterozyklus;
   n ist eine ganze Zahl von 1 bis 10;
   R4 ist Wasserstoff, Alkyl oder Aryl; oder
   R4 steht für -(CH₂)ₓ-N(R41)(R42), wobei gilt:
      R41 und R42 sind unabhängig voneinander Wasserstoff, Alkyl oder Aryl; oder
      R41 und R42 bilden gemeinsam mit dem sie tragenden N-Atom einen aliphatischen, ungesättigten oder
      aromatischen Heterozyklus;
      x ist eine ganze Zahl von 1 bis 10;
      und/oder:
(B) Amine der allgemeinen Formel (3): 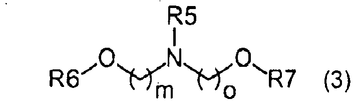 wobei gilt:
   R5 ist Wasserstoff, Alkyl oder Aryl;
   R6 und R7 sind unabhängig voneinander Wasserstoff, Alkyl oder Aryl;
   m und o sind unabhängig voneinander eine ganze Zahl von 1 bis 10.

Der in Schritt 2. des Verfahrens optional einsetzbare Katalysator kann ebenfalls aus den beschriebenen Gruppen (A) und/oder (B) ausgewählt sein, und/oder kann ausgewählt sein aus der Gruppe (C): Diazabicyclo[2.2.2]octan, Diazabicyclo[5.4.0]undec-7-en, Dialkylbenzylamin, Dimethylpiperazin, 2,2'-Dimorpholinyldiethylether und/oder Pyridin.

Amine der allgemeinen Formel (2) können im weitesten Sinne als Aminoalkohole oder deren Ether beschrieben werden. Ist R4 Wasserstoff, so sind die Katalysatoren in eine Polyurethanmatrix einbaubar, wenn das Polyetherpolyol mit einem Polyisocyanat umgesetzt wird. Dieses ist vorteilhaft, um das Austreten des Katalysators, der im Falle von Aminen mit nachteiligen Geruchsproblemen einhergehen kann, an die Polyurethanoberfläche, die sogenannte "fogging"-oder VOC (volatile organic compounds)-Problematik, zu verhindern.

Amine der allgemeinen Formel (3) können im weitesten Sinne als Amino(bis)alkohole oder deren Ether beschrieben werden. Sind R6 oder R7 Wasserstoff, so sind diese Katalysatoren ebenfalls in eine Polyurethanmatrix einbaubar.

Es ist bevorzugt, dass in dem Amin der allgemeinen Formel (2) R2 und R3 Methyl sind, R4 Wasserstoff ist und n = 2 ist oder aber R2 und R3 Methyl sind, R4 -(CH₂)₂-N(CH₃)₂ ist und n = 2 ist. Insgesamt ergibt sich also entweder N,N-Dimethylethanolamin oder Bis(2-(dimethylamino)ethyl)ether.

Es ist weiterhin bevorzugt, dass in dem Amin der allgemeinen Formel (3) R5 Methyl ist, R6 und R7 Wasserstoff sind, m = 2 ist und o = 2 ist. Insgesamt ergibt sich also N-Methyldiethanolamin.

In einer weiteren Ausführungsform des erfindungsgemäßen Verfahrens beträgt in Schritt 2. das molare Verhältnis von zyklischem Anhydrid zu Hydroxylgruppen in dem in Schritt 1. erhaltenen Produkt ≥ 0,75 : 1 bis ≤ 1,3 : 1. Vorzugsweise beträgt das Verhältnis ≥ 0,95 : 1 bis ≤ 1,25 : 1, mehr bevorzugt ≥ 1,02 : 1 bis ≤ 1,15 : 1.

In einer weiteren Ausführungsform des erfindungsgemäßen Verfahrens liegt in Schritt 3. der Katalysator, wie in Anspruch 1 definiert, welcher pro Molekül mindestens ein Stickstoffatom umfasst, bezogen auf die Gesamtmasse des Reaktionsansatzes in Schritt 3. in einem Anteil von ≥ 500 ppm bis ≤ 1500 ppm vor. Der Anteil des Katalysators kann auch ≥ 750 ppm bis ≤ 1250 ppm betragen. Gleiches gilt entsprechend, wenn in Schritt 2. auch ein solcher Katalysator eingesetzt wird.

In einer weiteren Ausführungsform des erfindungsgemäßen Verfahrens beträgt in Schritt 3. das molare Verhältnis von Ethylenoxid zu Hydroxylgruppen in dem in Schritt 1. erhaltenen Produkt ≥ 0,90 : 1 bis ≤ 5,0 : 1. Das Verhältnis kann auch ≥ 1,0 : 1 bis ≤ 2,0 : 1 oder vorzugsweise ≥ 1,05 : 1 bis ≤ 1,2 : 1 betragen.

Gegenstand der vorliegenden Erfindung ist weiterhin ein Polyetherpolyol mit primären Hydroxyl-Endgruppen, erhältlich durch ein erfindungsgemäßes Verfahren und umfassend einen Polyetherblock, eine endständige Hydroxyethylgruppe sowie eine den Polyetherblock und die endständige Hydroxyethylgruppe verbindende Diester-Einheit und wobei der molare Anteil an endständigen Doppelbindungen, bezogen auf alle Endgruppen des Polyetherpolyols, ≥ 0 Milliäquivalent pro kg bis ≤ 10 Milliäquivalent pro kg beträgt. Das Polyetherpolyol ist durch ein erfindungsgemäßes Verfahren erhältlich und wird insbesondere hierdurch erhalten. Daher wird zu Details seines Aufbaus auf die Ausführungen zum Verfahren verwiesen.

Der Polyetherblock kann beispielsweise, ohne hierauf beschränkt zu sein, ein auf einem di-, tri-, tetra- oder pentafunktionellen Alkohol gestarteter Ethylenoxidblock, Propylenoxidblock, Ethylenoxid/Propylenoxid-Mischblock und/oder eine beliebige Abfolge dieser Blöcke sein. Die Anzahl der Monomereinheiten im Polyetherblock, also zum Beispiel die Anzahl der Ethylenoxid-oder Propylenoxid-Einheiten, kann in einem Bereich von ≥ 10 Monomereinheiten bis ≤ 5000 Monomereinheiten, vorzugsweise von ≥ 50 Monomereinheiten bis ≤ 1000 Monomereinheiten liegen.

An den Polyetherblock schließt sich eine Diester-Einheit an, welche auf das Produkt der Reaktion einer OH-Endgruppe des Polyetherblocks mit einem zyklischen Carbonsäureanhydrid zurückgeführt werden kann. Unter Ringöffnung wird zunächst ein Halbester gebildet, welcher anschließend mit Ethylenoxid zur Hydroxyethyl-Endgruppe reagiert. Beispiele für das zyklische Carbonsäureanhydrid sind Phthalsäureanhydrid, Tetrahydrophthalsäureanhydrid, Bernsteinsäureanhydrid und/oder Maleinsäureanhydrid.

Das erfindungsgemäße Polyetherpolyol zeichnet sich dadurch aus, dass der Anteil an endständigen Doppelbindungen, bezogen auf alle Endgruppen des Polyetherpolyols (wobei hier die Gesamtheit der Polyetherpolyol-Moleküle zu verstehen ist) molmassenunabhängig im Bereich von ≥ 0 bis ≤ 10 Milliäquivalenten pro kg liegt. Für alle praktischen Zwecke ist er also frei von ungesättigten Endgruppen. Diese Endgruppen würden zu einer Minderfunktionalität des Polyethers führen und entsprechende Nachteile bei der Herstellung von Polyurethan-Polymeren mit sich bringen. Man vermeidet die endständigen Doppelbindungen beispielsweise dadurch, dass der Polyetherblock mittels DMC-Katalyse auf den Starter-Alkohol aufpolymerisiert wird. Man kann das erfindungsgemäße Polyetherpolyol mittels ¹H-NMR-Spektroskopie auf das Fehlen der ungesättigten Endgruppen untersuchen. Eine weitere gebräuchliche Methode ist die Bestimmung der endständigen Doppelbindungen mittels Quecksilberacetat gemäß ISO 17710. Der Gehalt kann auch ≥ 0 Milliäquivalent pro kg bis ≤ 5 Milliäquivalent pro kg betragen. Erfindungsgemäße Polyetherpolyole können weiterhin Funktionalitäten im Bereich von ≥ 2 bis ≤ 6 und Molmassen im Bereich von ≥ 1800 Da bis ≤ 20000 Da aufweisen.

In einer Ausführungsform des erfindungsgemäßen Polyetherpolyols beträgt der molare Anteil von primären Hydroxylgruppen ≥ 50 mol-% bis ≤ 100 mol-%. Hierunter ist der molare Anteil von primären Hydroxylgruppen gegenüber sekundären Hydroxylgruppen in dem Polyetherpolyol insgesamt, also nicht auf ein einzelnes Molekül bezogen, zu verstehen. Er lässt sich zum Beispiel mittels ¹H-NMR-Spektroskopie bestimmen. Der Anteil kann auch in einem Bereich von ≥ 55 mol-% bis ≤ 90 mol-% oder von ≥ 60 mol-% bis ≤ 85 mol-% liegen.

In einer weiteren Ausführungsform des erfindungsgemäßen Polyetherpolyols weist dieses eine OH-Zahl von ≥ 10 mg KOH/g bis ≤ 100 mg KOH/g auf. Die Hydroxylzahl lässt sich anhand der Norm DIN 53240 bestimmen und kann auch ≥ 15 mg KOH/g bis ≤ 80 mg KOH/g oder ≥ 20 mg KOH/g bis ≤ 50 mg KOH/g betragen.

In einer weiteren Ausführungsform des erfindungsgemäßen Polyetherpolyols weist dieses eine Säurezahl von ≥ 0,01 mg KOH/g bis ≤ 5 mg KOH/g auf. Die Säurezahl lässt sich anhand der Norm DIN 53402 bestimmen und kann auch ≥ 0,02 mg KOH/g bis ≤ 4,9 mg KOH/g oder ≥ 0,02 mg KOH/g bis ≤ 4,8 mg KOH/g betragen.

Ein weiterer Gegenstand der vorliegenden Erfindung ist eine Polyetherpolyol-Zusammensetzung, umfassend ein erfindungsgemäßes Polyetherpolyol sowie weiterhin:
(A) Amine der allgemeinen Formel (4): 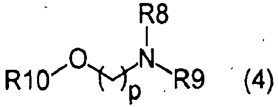 wobei gilt:
   R8 und R9 sind unabhängig voneinander Wasserstoff, Alkyl oder Aryl; oder
   R8 und R9 bilden gemeinsam mit dem sie tragenden N-Atom einen aliphatischen, ungesättigten oder aromatischen Heterozyklus;
   p ist eine ganze Zahl von 1 bis 10, also 1, 2, 3, 4, 5, 6, 7, 8, 9 oder 10;
   R10 ist Wasserstoff, Alkyl oder Aryl; oder
   R10 steht für -(CH₂)_{y}-N(R₁₁)(R₁₂), wobei gilt:
      R11 und R12 sind unabhängig voneinander Wasserstoff, Alkyl oder Aryl; oder
      R11 und R12 bilden gemeinsam mit dem sie tragenden N-Atom einen aliphatischen, ungesättigten oder aromatischen Heterozyklus;
      y ist eine ganze Zahl von 1 bis 10, also 1, 2, 3, 4, 5, 6, 7, 8, 9 oder 10;
(B) Amine der allgemeinen Formel (5): 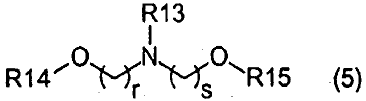 wobei gilt:
   R13 ist Wasserstoff, Alkyl oder Aryl;
   R14 und R15 sind unabhängig voneinander Wasserstoff, Alkyl oder Aryl;
   r und s sind unabhängig voneinander eine ganze Zahl von 1 bis 10, also 1, 2, 3, 4, 5, 6, 7, 8, 9 oder 10;
      und/oder:
(C) Diazabicyclo[2.2.2]octan, Diazabicyclo[5.4.0]undec-7-en, Dialkylbenzylamin, Dimethylpiperazin, 2,2'-Dimorpholinyldiethylether und/oder Pyridin.

Solche Verbindungen können in bestimmten Varianten auch als sogenannte Treibkatalysatoren verwendet werden, das heißt, sie katalysieren bevorzugt die Reaktion der Isocyanatgruppen mit Wasser unter Bildung von Kohlendioxid, in geringerem Ausmaß auch deren Reaktion mit Hydroxylgruppen unter Bildung von Urethangruppen. Daher kann diese Zusammensetzung unmittelbar weiter in der Herstellung von Polyurethanen eingesetzt werden. Wenn Zerewitinoffaktive Wasserstoffatome vorhanden sind, können diese Katalysatoren in eine Polyurethanmatrix eingebaut werden. Dieses verringert den Gehalt an flüchtigen organischen Substanzen im Polyurethan. Bevorzugt sind N,N-Dimethylethanolamin, Bis(2-(dimethylamino)ethyl)ether oder N-Methyldiethanolamin.

Die Massenanteil dieser Verbindungen (A), (B) und/oder (C) kann relativ zum erfindungsgemäßen Polyol beispielsweise ≥ 10 ppm bis ≤ 10000 ppm, bevorzugt ≥ 50 ppm bis ≤ 5000 ppm und mehr bevorzugt ≥ 100 ppm bis ≤ 2000 ppm betragen.

Ein weiterer Gegenstand der vorliegenden Erfindung ist ein Polyurethanpolymer, erhältlich aus der Reaktion eines Polyisocyanats mit einem erfindungsgemäßen Polyetherpolyol oder einer erfindungsgemäßen Polyetherpolyol-Zusammensetzung. Erfindungsgemäß mit eingeschlossen unter dem Begriff "Polyurethanpolymer" sind auch Prepolymere, die aus der Reaktion eines Polyisocyanats mit einem erfindungsgemäßen Polyether oder einer erfindungsgemäßen Polyetherpolyol-Zusammensetzung erhältlich sind.

Die vorliegende Erfindung wird anhand der nachfolgenden Beispiele weiter erläutert. Hierbei haben die verwendeten Materialien und Abkürzungen die folgende Bedeutung und Bezugsquellen:

| | |
|---|---|
| 2,2,2-Diazabicyclooctan (DABCO): | Aldrich |
| N,N-Dimethylethanolamin (DMEA): | Aldrich |
| Bis(2-(dimethylamino)ethyl)ether (DMAEE): | Alfa Aesar |
| Triethylamin: | Aldrich |
| Tetrahydrophthalsäureanhydrid (THPA): | Aldrich |

Die Analysen wurden wie folgt durchgeführt:
   - Viskosität:: Rheometer MCR 51 der Firma Anton Paar
   Bestimmung des molaren Anteils der primären OH-Gruppen: mittels ¹H-NMR (Bruker DPX 400, Deuterochloroform)
   - Hydroxylzahl:: anhand der Norm DIN 53240
   - Säurezahl:: anhand der Norm DIN 53402

### 1. Herstellung der DMC-katalysierten Vorstufen:

### Vorstufe A:

In einem 1 Liter-Edelstahldruckreaktor wurden 117,6 g eines Poly(oxypropylen)triols mit einer OH-Zahl von 238 mg KOH/g und 0,024 g DMC-Katalysator (hergestellt gemäß EP 0 700 949 A2) unter Stickstoff vorgelegt, auf 130 °C aufgeheizt und dann für 30 Minuten bei 0,1 bar unter Durchleiten von Stickstoff gestrippt. Anschließend wurden 682 g Propylenoxid bei 130 °C innerhalb von 3 Stunden zudosiert. Nach einer Nachreaktionszeit bei 130 °C bis zur Druckkonstanz im Reaktor wurden leichtflüchtige Anteile bei 90 °C für 30 Minuten im Vakuum abdestilliert und das Reaktionsgemisch anschließend auf Raumtemperatur abgekühlt. Die OH-Zahl des Produkts lag bei 34,1 mg KOH/g bei einer Viskosität (25 °C) von 967 mPas.

### Vorstufe B:

In einem 1 Liter-Edelstahldruckreaktor wurden 117,6 g eines Poly(oxypropylen)triols mit einer OH-Zahl von 238 mg KOH/g und 0,024 g DMC-Katalysator (hergestellt gemäß EP 0 700 949 A2) unter Stickstoff vorgelegt, auf 130 °C aufgeheizt und dann für 30 Minuten bei 0,1 bar unter Durchleiten von Stickstoff gestrippt. Dann wurde bei 130 °C innerhalb von 3 Stunden zunächst ein Gemisch von 504 g Propylenoxid und 38 g Ethylenoxid, anschließend ein Gemisch von 53 g Propylenoxid und 17 g Ethylenoxid und schließlich ein Gemisch von 35 g Propylenoxid und 35 g Ethylenoxid zudosiert. Nach einer Nachreaktionszeit bei 130 °C bis zur Druckkonstanz im Reaktor wurden leichtflüchtige Anteile bei 90 °C für 30 Minuten im Vakuum abdestilliert und das Reaktionsgemisch anschließend auf Raumtemperatur abgekühlt. Die OH-Zahl des Produkts lag bei 34,6 mg KOH/g bei einer Viskosität (25 °C) von 954 mPas.

### Vorstufe C:

In einem 1 Liter-Edelstahldruckreaktor wurden 117,6 g eines Poly(oxypropylen)triols mit einer OH-Zahl von 238 mg KOH/g und 0,024 g DMC-Katalysator (hergestellt gemäß EP 0 700 949 A2) unter Stickstoff vorgelegt, auf 130 °C aufgeheizt und dann für 30 Minuten bei 0,1 bar unter Durchleiten von Stickstoff gestrippt. Dann wurde bei 130 °C innerhalb von 3 Stunden zunächst ein Gemisch von 439 g Propylenoxid und 33 g Ethylenoxid, dann ein Gemisch von 53 g Propylenoxid und 17 g Ethylenoxid, anschließend ein Gemisch von 35 g Propylenoxid und 35 g Ethylenoxid und schließlich ein Gemisch von 21 g Propylenoxid und 49 g Ethylenoxid zudosiert. Nach einer Nachreaktionszeit bei 130 °C bis zur Druckkonstanz im Reaktor wurden leichtflüchtige Anteile bei 90°C für 30 Minuten im Vakuum abdestilliert und das Reaktionsgemisch anschließend auf Raumtemperatur abgekühlt. Die OH-Zahl des Produkts lag bei 35,3 mg KOH/g bei einer Viskosität (25°C) von 916 mPas.

Die nachfolgende Tabelle 1 fasst die Daten zu den Vorstufen A, B und C zusammen.

| | | Vorstufe A | Vorstufe B | Vorstufe C |
|---|---|---|---|---|
| Polyoxypropylentriol | [g] | 117,6 | 117,6 | 117,6 |
| DMC-Katalysator | [g] | 0,024 | 0,024 | 0,024 |
| Propylen-/Ethylenoxid | [g / g] | 682/0 | 504 / 38 | 439 / 33 |
| Propylen-/Ethylenoxid | [g / g] | | 53 / 17 | 53 / 17 |
| Propylen-/Ethylenoxid | [g / g] | | 35 / 35 | 35 / 35 |
| Propylen-/Ethylenoxid | [g / g] | | | 21 / 49 |
| Anteil an Ethylenoxid im insgesamt eingesetzten Epoxid | [Gew.-%] | 0 | 15 | 24 |
| | | | | |
| OH-Zahl | [mg KOH/g] | 34,1 | 34,6 | 35,3 |
| Viskosität | [mPas, 25 °C] | 967 | 954 | 916 |

### 2. Umsetzung der DMC-katalysierten Vorstufen mit zyklischen Anhydriden und Ethylenoxid unter Amin-Katalyse:

### Beispiel 1 (Vergleichsbeispiel):

In einem 1 Liter-Edelstahldruckreaktor wurden 400 g der DMC-katalysierten Vorstufe A, 40,66 g Tetrahydrophthalsäureanhydrid und 0,462 g (1000 ppm, bezogen auf den Gesamtansatz) Triethylamin unter Stickstoff vorgelegt. Das molare Verhältnis zwischen Anhydrid und den Hydroxylgruppen der Vorstufe A betrug 1,1 / 1. Dann wurde auf 125 °C aufgeheizt und für 3 Stunden bei dieser Temperatur gerührt. Anschließend wurden über einen Zeitraum von 30 Minutenuten 21,42 g Ethylenoxid bei 125 °C in den Reaktor dosiert. Das molare Verhältnis zwischen Ethylenoxid und den Hydroxylgruppen der Vorstufe A betrug 2 / 1. Nach einer Nachreaktionszeit bei 125 °C bis zur Druckkonstanz im Reaktor wurden leichtflüchtige Anteile bei 90 °C für 30 Minuten im Vakuum abdestilliert und das Reaktionsgemisch anschließend auf Raumtemperatur abgekühlt. Die Säurezahl des Produkts lag bei 23,6 mg KOH/g bei einer Viskosität (25 °C) von 4140 mPas. Die sehr hohe Säurezahl zeigt, dass nur ein geringer Umsatz mit Ethylenoxid stattgefunden hat.

### Beispiel 2: (Vergleichsbeispiel):

In einem 1 Liter-Edelstahldruckreaktor wurden 400 g der DMC-katalysierten Vorstufe A, 40,66 g Tetrahydrophthalsäureanhydrid und 0,462 g (1000 ppm, bezogen auf den Gesamtansatz) DABCO (Triethylendiamin) unter Stickstoff vorgelegt. Das molare Verhältnis zwischen Anhydrid und den Hydroxylgruppen der Vorstufe A betrug 1,1 / 1. Dann wurde auf 125 °C aufgeheizt und für 3 Stunden bei dieser Temperatur gerührt. Anschließend wurden über einen Zeitraum von 30 Minuten 21,42 g Ethylenoxid bei 125 °C in den Reaktor dosiert. Das molare Verhältnis zwischen Ethylenoxid und den Hydroxylgruppen der Vorstufe A betrug 2 / 1. Nach einer Nachreaktionszeit bei 125 °C bis zur Druckkonstanz im Reaktor wurden leichtflüchtige Anteile bei 90 °C für 30 Minuten im Vakuum abdestilliert und das Reaktionsgemisch anschließend auf Raumtemperatur abgekühlt.

Produkteigenschaften:

| | |
|---|---|
| OH-Zahl: | 28,2 mg KOH/g |
| Säurezahl: | 2,58 mg KOH/g |
| Viskosität (25 °C): | 3035 mPas |
| Primäre OH-Gruppen: | 65 % |

### Beispiel 3:

In einem 1 Liter-Edelstahldruckreaktor wurden 400 g der DMC-katalysierten Vorstufe A, 40,66 g Tetrahydrophthalsäureanhydrid und 0,462 g (1000 ppm, bezogen auf den Gesamtansatz) Bis(2-dimethylaminoethyl)ether unter Stickstoff vorgelegt. Das molare Verhältnis zwischen Anhydrid und den Hydroxylgruppen der Vorstufe A betrug 1,1 / 1. Dann wurde auf 125 °C aufgeheizt und für 3 Stunden bei dieser Temperatur gerührt. Anschließend wurden über einen Zeitraum von 30 Minuten 21,42 g Ethylenoxid bei 125 °C in den Reaktor dosiert. Das molare Verhältnis zwischen Ethylenoxid und den Hydroxylgruppen der Vorstufe A betrug 2 / 1. Nach einer Nachreaktionszeit bei 125 °C bis zur Druckkonstanz im Reaktor wurden leichtflüchtige Anteile bei 90 °C für 30 Minuten im Vakuum abdestilliert und das Reaktionsgemisch anschließend auf Raumtemperatur abgekühlt.

Produkteigenschaften:

| | |
|---|---|
| OH-Zahl: | 32,8 mg KOH/g |
| Säurezahl: | 0,04 mg KOH/g |
| Viskosität (25 °C): | 2685 mPas |
| Primäre OH-Gruppen: | 69 % |

### Beispiel 4:

In einem 1 Liter-Edelstahldruckreaktor wurden 400 g der DMC-katalysierten Vorstufe B, 37,54 g Tetrahydrophthalsäureanhydrid und 0,459 g (1000 ppm, bezogen auf den Gesamtansatz) Bis(2-dimethylaminoethyl)ether unter Stickstoff vorgelegt. Das molare Verhältnis zwischen Anhydrid und den Hydroxylgruppen der Vorstufe B betrug 1 / 1. Dann wurde auf 125 °C aufgeheizt und für 3 Stunden bei dieser Temperatur gerührt. Anschließend wurden über einen Zeitraum von 30 Minuten 21,74 g Ethylenoxid bei 105°C in den Reaktor dosiert. Das molare Verhältnis zwischen Ethylenoxid und den Hydroxylgruppen der Vorstufe B betrug 2 / 1. Nach einer Nachreaktionszeit bei 105°C bis zur Druckkonstanz im Reaktor wurden leichtflüchtige Anteile bei 90 °C für 30 Minuten im Vakuum abdestilliert und das Reaktionsgemisch anschließend auf Raumtemperatur abgekühlt.

Produkteigenschaften:

| | |
|---|---|
| OH-Zahl: | 32,8 mg KOH/g |
| Säurezahl: | 0,05 mg KOH/g |
| Viskosität (25 °C): | 2437 mPas |
| Primäre OH-Gruppen: | 73 % |

### Beispiel 5:

In einem 1 Liter-Edelstahldruckreaktor wurden 400 g der DMC-katalysierten Vorstufe B, 37,54 g Tetrahydrophthalsäureanhydrid und 0,459 g (1000 ppm, bezogen auf den Gesamtansatz) Bis(2-dimethylaminoethyl)ether unter Stickstoff vorgelegt. Das molare Verhältnis zwischen Anhydrid und den Hydroxylgruppen der Vorstufe B betrug 1 / 1. Dann wurde auf 125 °C aufgeheizt und für 3 Stunden bei dieser Temperatur gerührt. Anschließend wurden über einen Zeitraum von 30 Minuten 21,74 g Ethylenoxid bei 145°C in den Reaktor dosiert. Das molare Verhältnis zwischen Ethylenoxid und den Hydroxylgruppen der Vorstufe B betrug 2 / 1. Nach einer Nachreaktionszeit bei 145°C bis zur Druckkonstanz im Reaktor wurden leichtflüchtige Anteile bei 90 °C für 30 Minuten im Vakuum abdestilliert und das Reaktionsgemisch anschließend auf Raumtemperatur abgekühlt.

Produkteigenschaften:

| | |
|---|---|
| OH-Zahl: | 32,1 mg KOH/g |
| Säurezahl: | 0,66 mg KOH/g |
| Viskosität (25 °C): | 2229 mPas |
| Primäre OH-Gruppen: | 69 % |

### Beispiel 6:

In einem 1 Liter-Edelstahldruckreaktor wurden 400 g der DMC-katalysierten Vorstufe B, 41,29 g Tetrahydrophthalsäureanhydrid und 0,463 g (1000 ppm, bezogen auf den Gesamtansatz) Bis(2-dimethylaminoethyl)ether unter Stickstoff vorgelegt. Das molare Verhältnis zwischen Anhydrid und den Hydroxylgruppen der Vorstufe B betrug 1,1 / 1. Dann wurde auf 125 °C aufgeheizt und für 3 Stunden bei dieser Temperatur gerührt. Anschließend wurden über einen Zeitraum von 30 Minuten 21,74 g Ethylenoxid bei 105°C in den Reaktor dosiert. Das molare Verhältnis zwischen Ethylenoxid und den Hydroxylgruppen der Vorstufe B betrug 2 / 1. Nach einer Nachreaktionszeit bei 105°C bis zur Druckkonstanz im Reaktor wurden leichtflüchtige Anteile bei 90 °C für 30 Minuten im Vakuum abdestilliert und das Reaktionsgemisch anschließend auf Raumtemperatur abgekühlt.

Produkteigenschaften:

| | |
|---|---|
| OH-Zahl: | 32,2 mg KOH/g |
| Säurezahl: | 0,53 mg KOH/g |
| Viskosität (25 °C): | 2750 mPas |
| Primäre OH-Gruppen: | 74% |

### Beispiel 7:

In einem 1 Liter-Edelstahldruckreaktor wurden 400 g der DMC-katalysierten Vorstufe B, 41,29 g Tetrahydrophthalsäureanhydrid und 0,463 g (1000 ppm, bezogen auf den Gesamtansatz) Bis(2-dimethylaminoethyl)ether unter Stickstoff vorgelegt. Das molare Verhältnis zwischen Anhydrid und den Hydroxylgruppen der Vorstufe B betrug 1,1 / 1. Dann wurde auf 125 °C aufgeheizt und für 3 Stunden bei dieser Temperatur gerührt. Anschließend wurden über einen Zeitraum von 30 Minuten 21,74 g Ethylenoxid bei 145°C in den Reaktor dosiert. Das molare Verhältnis zwischen Ethylenoxid und den Hydroxylgruppen der Vorstufe B betrug 2 / 1. Nach einer Nachreaktionszeit bei 145°C bis zur Druckkonstanz im Reaktor wurden leichtflüchtige Anteile bei 90 °C für 30 Minuten im Vakuum abdestilliert und das Reaktionsgemisch anschließend auf Raumtemperatur abgekühlt.

Produkteigenschaften:

| | |
|---|---|
| OH-Zahl: | 28,3 mg KOH/g |
| Säurezahl: | 2,84 mg KOH/g |
| Viskosität (25 °C): | 2525 mPas |
| Primäre OH-Gruppen: | 66 % |

### Beispiel 8:

In einem 1 Liter-Edelstahldruckreaktor wurden 400 g der DMC-katalysierten Vorstufe B, 41,29 g Tetrahydrophthalsäureanhydrid und 0,463 g (1000 ppm, bezogen auf den Gesamtansatz) Bis(2-dimethylaminoethyl)ether unter Stickstoff vorgelegt. Das molare Verhältnis zwischen Anhydrid und den Hydroxylgruppen der Vorstufe B betrug 1,1 / 1. Dann wurde auf 125 °C aufgeheizt und für 3 Stunden bei dieser Temperatur gerührt. Anschließend wurden über einen Zeitraum von 30 Minuten 21,74 g Ethylenoxid bei 90°C in den Reaktor dosiert. Das molare Verhältnis zwischen Ethylenoxid und den Hydroxylgruppen der Vorstufe B betrug 2 / 1. Nach einer Nachreaktionszeit bei 90°C bis zur Druckkonstanz im Reaktor wurden leichtflüchtige Anteile bei 90 °C für 30 Minuten im Vakuum abdestilliert und das Reaktionsgemisch anschließend auf Raumtemperatur abgekühlt.

Produkteigenschaften:

| | |
|---|---|
| OH-Zahl: | 28,2 mg KOH/g |
| Säurezahl: | 2,41 mg KOH/g |
| Viskosität (25 °C): | 3074 mPas |
| Primäre OH-Gruppen: | 75 % |

### Beispiel 9:

In einem 1 Liter-Edelstahldruckreaktor wurden 200 g der DMC-katalysierten Vorstufe B, 20,64 g Tetrahydrophthalsäureanhydrid und 0,232 g (1000 ppm, bezogen auf den Gesamtansatz) Bis(2-dimethylaminoethyl)ether unter Stickstoff vorgelegt. Das molare Verhältnis zwischen Anhydrid und den Hydroxylgruppen der Vorstufe B betrug 1,1 / 1. Dann wurde auf 125 °C aufgeheizt und für 3 Stunden bei dieser Temperatur gerührt. Anschließend wurden über einen Zeitraum von 30 Minuten 6,0 g Ethylenoxid bei 125 °C in den Reaktor dosiert. Das molare Verhältnis zwischen Ethylenoxid und den Hydroxylgruppen der Vorstufe B betrug 1,1 / 1. Nach einer Nachreaktionszeit bei 125 °C bis zur Druckkonstanz im Reaktor wurden leichtflüchtige Anteile bei 90 °C für 30 Minuten im Vakuum abdestilliert und das Reaktionsgemisch anschließend auf Raumtemperatur abgekühlt.

Produkteigenschaften:

| | |
|---|---|
| OH-Zahl: | 24,1 mg KOH/g |
| Säurezahl: | 4,75 mg KOH/g |
| Viskosität (25 °C): | 2578 mPas |
| Primäre OH-Gruppen: | 63 % |

### Beispiel 10: (Vergleichsbeispiel):

In einem 1 Liter-Edelstahldruckreaktor wurden 300 g der DMC-katalysierten Vorstufe C, 31,59 g Tetrahydrophthalsäureanhydrid und 0,348 g (1000 ppm, bezogen auf den Gesamtansatz) DABCO (Triethylendiamin) unter Stickstoff vorgelegt. Das molare Verhältnis zwischen Anhydrid und den Hydroxylgruppen der Vorstufe C betrug 1,1 / 1. Dann wurde auf 125 °C aufgeheizt und für 3 Stunden bei dieser Temperatur gerührt. Anschließend wurden über einen Zeitraum von 30 Minuten 16,63 g Ethylenoxid bei 125 °C in den Reaktor dosiert. Das molare Verhältnis zwischen Ethylenoxid und den Hydroxylgruppen der Vorstufe C betrug 2 / 1. Nach einer Nachreaktionszeit bei 125 °C bis zur Druckkonstanz im Reaktor wurden leichtflüchtige Anteile bei 90 °C für 30 Minuten im Vakuum abdestilliert und das Reaktionsgemisch anschließend auf Raumtemperatur abgekühlt.

Produkteigenschaften:

| | |
|---|---|
| OH-Zahl: | 33,7 mg KOH/g |
| Säurezahl: | 0,23 mg KOH/g |
| Viskosität (25 °C): | 2760 mPas |
| Primäre OH-Gruppen: | 76 % |

### Beispiel 11:

In einem 1 Liter-Edelstahldruckreaktor wurden 300 g der DMC-katalysierten Vorstufe C, 31,59 g Tetrahydrophthalsäureanhydrid und 0,348 g (1000 ppm, bezogen auf den Gesamtansatz) N,N-Dimethylethanolamin unter Stickstoff vorgelegt. Das molare Verhältnis zwischen Anhydrid und 1 l Edelstahldruckreaktor den Hydroxylgruppen der Vorstufe C betrug 1,1 / 1. Dann wurde auf 125 °C aufgeheizt und für 3 Stunden bei dieser Temperatur gerührt. Anschließend wurden über einen Zeitraum von 30 Minuten 16,63 g Ethylenoxid bei 125 °C in den Reaktor dosiert. Das molare Verhältnis zwischen Ethylenoxid und den Hydroxylgruppen der Vorstufe C betrug 2 / 1. Nach einer Nachreaktionszeit bei 125 °C bis zur Druckkonstanz im Reaktor wurden leichtflüchtige Anteile bei 90 °C für 30 Minuten im Vakuum abdestilliert und das Reaktionsgemisch anschließend auf Raumtemperatur abgekühlt.

Produkteigenschaften:

| | |
|---|---|
| OH-Zahl: | 34,3 mg KOH/g |
| Säurezahl: | 0,12 mg KOH/g |
| Viskosität (25 °C): | 2274 mPas |
| Primäre OH-Gruppen: | 65 % |

### Beispiel 12:

In einem 1 Liter-Edelstahldruckreaktor wurden 300 g der DMC-katalysierten Vorstufe C, 31,59 g Tetrahydrophthalsäureanhydrid und 0,348 g (1000 ppm, bezogen auf den Gesamtansatz) Bis(2-dimethylaminoethyl)ether unter Stickstoff vorgelegt. Das molare Verhältnis zwischen Anhydrid und den Hydroxylgruppen der Vorstufe C betrug 1,1 / 1. Dann wurde auf 125 °C aufgeheizt und für 3 Stunden bei dieser Temperatur gerührt. Anschließend wurden über einen Zeitraum von 30 Minuten 16,63 g Ethylenoxid bei 125 °C in den Reaktor dosiert. Das molare Verhältnis zwischen Ethylenoxid und den Hydroxylgruppen der Vorstufe C betrug 2 / 1. Nach einer Nachreaktionszeit bei 125 °C bis zur Druckkonstanz im Reaktor wurden leichtflüchtige Anteile bei 90 °C für 30 Minuten im Vakuum abdestilliert und das Reaktionsgemisch anschließend auf Raumtemperatur abgekühlt.

Produkteigenschaften:

| | |
|---|---|
| OH-Zahl: | 34,6 mg KOH/g |
| Säurezahl: | 0,06 mg KOH/g |
| Viskosität (25 °C): | 2535 mPas |
| Primäre OH-Gruppen: | 70 % |

Die nachfolgenden Tabellen 2 fassen die Daten zu den Beispielen 1 bis 12 zusammen:

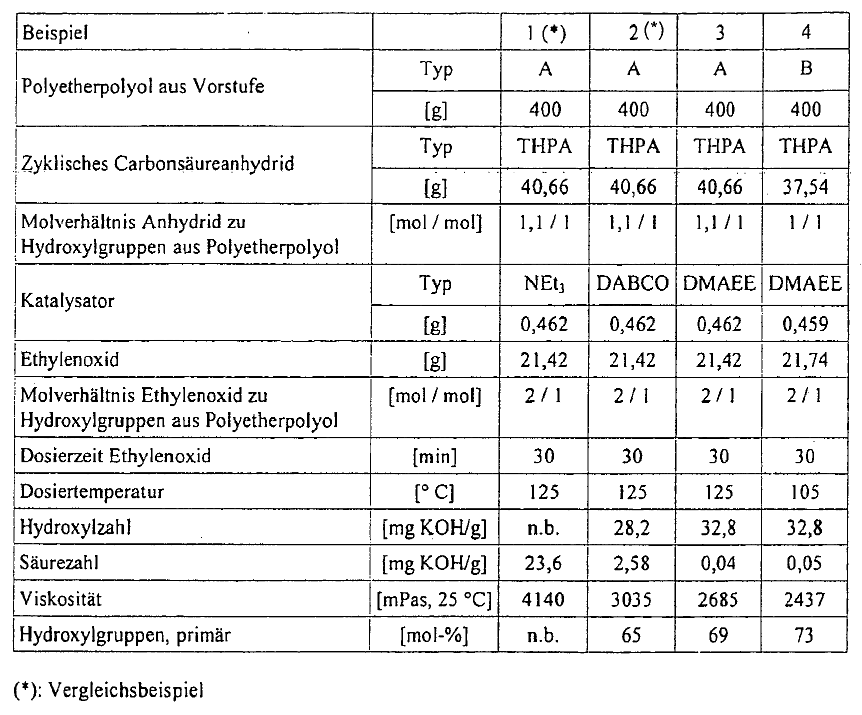

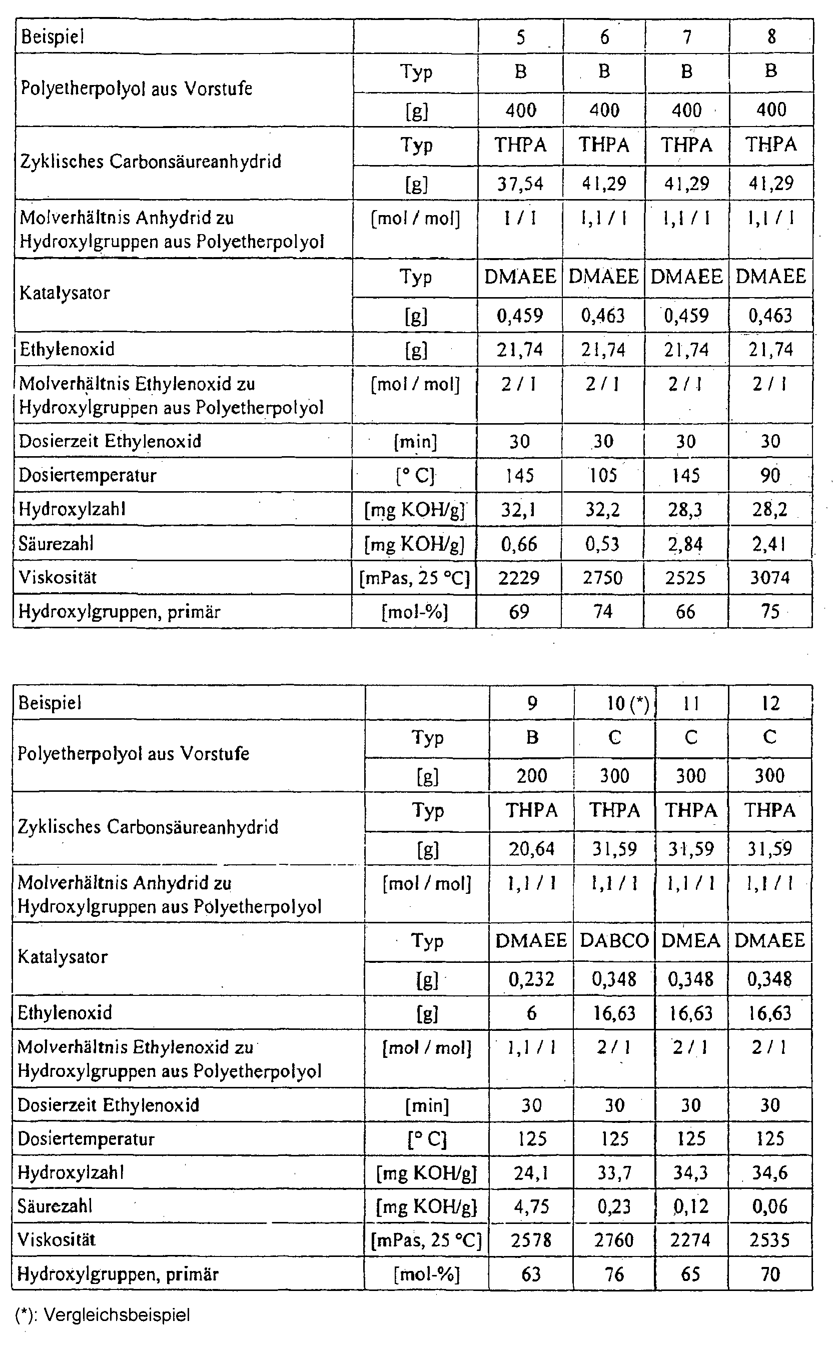

Anhand der sehr geringen Säurezahlen in den Endprodukten der erfindungsgemäßen Beispiele lässt sich erkennen, dass kaum freie Carboxylgruppen, welche nach der Öffnung des zyklischen Anhydrids entstehen, nicht mit Ethylenoxid reagiert haben. Ein Vergleich der Hydroxylzahlen der Endprodukte und der eingangs eingesetzten Vorstufen-Polyethern A, B und C zeigt weiterhin, dass eine nur geringe Molekulargewichtserhöhung der Vorstufen A, B und C durch das erfindungsgemäße Verfahren eingetreten ist. Schließlich kann man in den Ergebnissen ablesen, dass jeweils primäre Endgruppen in einem Anteil von über 50% erhalten wurden.

## Patentansprüche

1. Verfahren zur Herstellung von Polyetherpolyolen mit primären Hydroxyl-Endgruppen, umfassend die Schritte:
1. Reaktion einer aktive Wasserstoffatome aufweisenden Starterverbindung mit einem Epoxid der allgemeinen Formel (1): 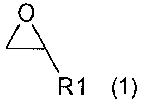 wobei R1 für Wasserstoff, einen Alkylrest oder einen Arylrest steht und mit der Maßgabe, dass ≥ 0 Gewichts-% bis ≤ 30 Gewichts-%, bezogen auf die Gesamtmenge des eingesetzten Epoxids (1), Ethylenoxid sind, wobei die Reaktion in Gegenwart eines Doppelmetallcyanid-Katalysators durchgeführt wird und wobei das Rohprodukt dieser Reaktion mit Ausnahme eines möglichen Destillationsschrittes keine weitere Aufreinigung erfährt;
2. Reaktion des in Schritt 1. erhaltenen Produkts mit einem zyklischen Carbonsäureanhydrid; und
3. Reaktion des in Schritt 2. erhaltenen Produkts mit Ethylenoxid in Gegenwart eines Katalysators, welcher pro Molekül mindestens ein Stickstoffatom umfasst, wobei nicht-zyklische, identisch substituierte tertiäre Amine ausgenommen sind, und
wobei der in Schritt 3. eingesetzte Katalysator ausgewählt ist aus der Gruppe umfassend:
(A) Amine der allgemeinen Formel (2): 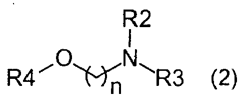 wobei gilt:
R2 und R3 sind unabhängig voneinander Wasserstoff, Alkyl oder Aryl; oder
R2 und R3 bilden gemeinsam mit dem sie tragenden N-Atom einen aliphatischen, ungesättigten oder aromatischen Heterozyklus;
n ist eine ganze Zahl von 1 bis 10;
R4 ist Wasserstoff. Alkyl oder Aryl; oder
R4 steht für -(CH₂)ₓ-N(R41)(R42), wobei gilt:
R41 und R42 sind unabhängig voreinander Wasserstoff, Alkyl oder Aryl; oder
R41 und R42 bilden gemeinsam mit dem sie tragenden N-Atom einen aliphatischen, ungesättigten oder aromatischen Heterozyklus;
x ist eine ganze Zahl von 1 bis 10;
und/oder:
(B) Amine der allgemeinen Formel (3): 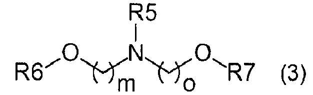 wobei gilt:
R5 ist Wasserstoff, Alkyl oder Aryl;
R6 und R7 sind unabhängig voneinander Wasserstoff, Alkyl oder Aryl;
m und o sind unabhängig voneinander eine ganze Zahl von 1 bis 10.

2. Verfahren gemäß Anspruch 1, wobei die in Schritt 1. eingesetzte Starterverbindung ein Poly(oxyalkylen)-Polyol mit einer durchschnittlichen Funktionalität von ≥ 2,0 bis ≤ 5,0, einem Zahlenmittel der Molekülmasse von ≥ 62 g/mol bis ≤ 1000 g/mol und einer OH-Zahl von ≥ 100 mg KOH/g bis ≤ 1860 mg KOH/g ist.

3. Verfahren gemäß Anspruch 1, wobei in dem Epoxid der allgemeinen Formel (1) R1 Wasserstoff, Methyl, Ethyl, n-Propyl, iso-Propyl, n-Butyl, sec-Butyl, iso-Butyl, tert-Butyl, Cyclohexyl und/oder Phenyl ist.

4. Verfahren gemäß Anspruch 1, wobei der Doppelmetallcyanid-Katalysator in Schritt 1. Zink, Kobalt und tert.-Butanol umfasst.

5. Verfahren gemäß Anspruch 1, wobei das in Schritt 2. eingesetzte zyklische Carbonsäureanhydrid ausgewählt ist aus der Gruppe umfassend Plithalsäureauhydrid, Tetrahydrophthalsäureanhydrid, Bernsteinsäureanhydrid und/oder Maleinsäureanhydrid.

6. Verfahren gemäß Anspruch 1, wobei in Schritt 2. das molare Verhältnis von zyklischem Anhydrid zu Hydroxylgruppen in dem in Schritt 1. erhaltenen Produkt ≥ 0,75 : 1 bis ≤ 1,3 : 1 beträgt.

7. Verfahren gemäß Anspruch 1, wobei in Schritt 3. der Katalysator, welcher pro Molekül mindestens ein Stickstoffatom umfasst, bezogen auf die Gesamtmasse des Reaktionsansatzes in Schritten 2. und 3. in einem Anteil von ≥ 500 ppm bis ≤ 1500 ppm vorliegt.

8. Verfahren gemäß Anspruch 1, wobei in Schritt 3. das molare Verhältnis von Ethylenoxid zu Hydroxylgruppen in dem in Schritt 1. erhaltenen Produkt ≥ 0,90 : 1 bis ≤ 5,0 : 1 beträgt.

9. Polyetherpolyol mit primären Hydroxyl-Endgruppen, erhältlich durch ein Verfahren gemäß Anspruch 1, umfassend einen Polyetherblock, eine endständige Hydroxyethylgruppe sowie eine den Polyetherblock und die endständige Hydroxyethylgruppe verbindende Diester-Einheit und wobei der molare Anteil an endständigen Doppelbindungen, bezogen auf alle Endgruppen des Polyetherpolyols, ≥ 0 Milliäquivalent pro kg bis ≤ 10 Milliäquivalent pro kg beträgt.

10. Polyetherpolyol gemäß Anspruch 9, wobei der molare Anteil von primären Hydroxylgruppen ≥ 50 mol-% bis ≤ 100 mol-% beträgt.

11. Polyetherpolyol gemäß Anspruch 9 mit einer OH-Zahl von ≥ 10 mg KOH/g bis ≤ 100 mg KOH/g.

12. Polyetherpolyol gemäß Anspruch 9 mit einer Säurezahl von ≥ 0,01 mg KOH/g bis ≤ 5 mg KOH/g.

13. Polyetherpolyol-Zusammensetzung, umfassend ein Polyetherpolyol gemäß Anspruch 9 sowie weiterhin:
(A) Amine der allgemeinen Formel (4): 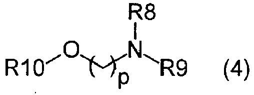 wobei gilt:
R8 und R9 sind unabhängig voneinander Wasserstoff, Alkyl oder Aryl; oder
R8 und R9 bilden gemeinsam mit dem sie tragenden N-Atom einen aliphatischen, ungesättigten oder aromatischen Heterozyklus;
p ist eine ganze Zahl von 1 bis 10, also 1, 2, 3, 4, 5, 6, 7, 8, 9 oder 10;
R10 ist Wasserstoff, Alkyl oder Aryl; oder
R10 steht für -(CH₂)_{y}-N(R11)(R12), wobei gilt:
R11 und R12 sind unabhängig voneinander Wasserstoff, Alkyl oder Aryl; oder
R11 und R12 bilden gemeinsam mit dem sie tragenden N-Atom einen aliphatischen, ungesättigten oder aromatischen Heterozyklus;
y ist eine ganze Zahl von 1 bis 10, also 1, 2, 3, 4, 5, 6, 7, 8, 9 oder 10;
(B) Amine der allgemeinen Formen (5): 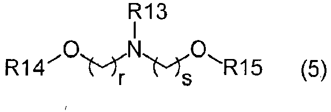 wobei gilt:
R13 ist Wasserstoff, Alkyl oder Aryl;
R14 und R15 sind unabhängig voneinander Wasserstoff, Alkyl oder Aryl;
r und s sind unabhängig voneinander eine ganze Zahl von 1 bis 10, also 1, 2, 3, 4, 5, 6, 7, 8, 9 oder 10;
und/oder:
(C) Diazabicyclo[2.2.2]octan, Diazabicyclo[5.4.0]undec-7-en, Dialkylbenzylamin, Dimethylpiperazin, 2,2'-Dimorpholinyldiethylether und/oder Pyridin.

14. Polyurethanpolymer, erhältlich aus der Reaktion eines Polyisocyanats mit einem Polyetherpolyol gemäß Anspruch 9 oder einer Polyetherpolyol-Zusammensetzung gemäß Anspruch 13.

## Claims

1. Process for producing polyether polyols having terminal primary hydroxyl groups, comprising the following steps:
1. Reaction of a starter compound having active hydrogen atoms with an epoxide of the general formula (1) : 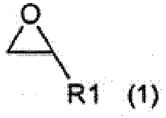 where R1 is hydrogen, an alkyl moiety or an aryl moiety, and with the proviso that from ≥ 0% by weight to ≤ 30% by weight, based on the total amount of the epoxide (1) used, are ethylene oxide,
where the reaction is carried out in the presence of a double-metal-cyanide catalyst, and where the crude product of the said reaction does not undergo any further purification with the exception of a possible distillation step;
2. Reaction of the product obtained in step 1. with a cyclic carboxylic anhydride; and
3. Reaction of the product obtained in step 2. with ethylene oxide in the presence of a catalyst which comprises at least one nitrogen atom per molecule, where non-cyclic, identically substituted tertiary amines are excluded, and
where the catalyst used in step 3. is one selected from the group consisting of:
(A) Amines of the general formula (2): 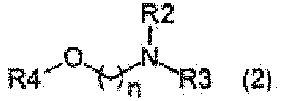 where:
R2 and R3 are mutually independently hydrogen, alkyl or aryl; or
R2 and R3 form, together with the N atom bearing them, an aliphatic, unsaturated or aromatic heterocycle;
n is an integer from 1 to 10;
R4 is hydrogen, alkyl or aryl; or
R4 is -(CH₂)ₓ-N(R41)(R42), where:
R41 and R42 are mutually independently hydrogen, alkyl or aryl; or
R41 and R42 form, together with the N atom bearing them, an aliphatic, unsaturated or aromatic heterocycle;
x is an integer from 1 to 10;
and/or:
(B) Amines of the general formula (3): 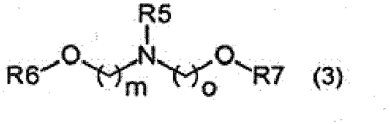 where:
R5 is hydrogen, alkyl or aryl;
R6 and R7 are mutually independently hydrogen, alkyl or aryl;
m and o are mutually independently an integer from 1 to 10.

2. Process according to Claim 1, where the starter compound used in step 1. is a poly(oxyalkylene) polyol with an average functionality of from ≥ 2.0 to ≤ 5.0, a number-average molar mass of from ≥ 62 g/mol to ≤ 1000 g/mol and with an OH number of from ≥ 100 mg KOH/g to ≤ 1860 mg KOH/g.

3. Process according to Claim 1, where R1 in the epoxide of the general formula (1) is hydrogen, methyl, ethyl, n-propyl, isopropyl, n-butyl, sec-butyl, isobutyl, tert-butyl, cyclohexyl and/or phenyl.

4. Process according to Claim 1, where the double-metal-cyanide catalyst in step 1. comprises zinc, cobalt and tert-butanol.

5. Process according to Claim 1, where the cyclic carboxylic anhydride used in step 2. is one selected from the group consisting of phthalic anhydride, tetrahydrophthalic anhydride, succinic anhydride and/or maleic anhydride.

6. Process according to Claim 1, where in step 2., the molar ratio of cyclic anhydride to hydroxy groups in the product obtained in step 1. is from ≥ 0.75 : 1 to ≤ 1.3 : 1.

7. Process according to Claim 1, where, in step 3., the proportion present of the catalyst which comprises at least one nitrogen atom per molecule is from ≥ 500 ppm to ≤ 1500 ppm, based on the total composition of the reaction mixture in steps 2. and 3.

8. Process according to Claim 1, where, in step 3., the molar ratio of ethylene oxide to hydroxy groups in the product obtained in step 1. is from ≥ 0.90 : 1 to ≤ 5.0 :1.

9. Polyether polyol having terminal primary hydroxy groups, obtainable via a process according to Claim 1, comprising a polyether block, a terminal hydroxyethyl group, and also a diester unit linking the polyether block and the terminal hydroxyethyl group and where the molar proportion of terminal double bonds, based on all terminal groups of the polyether polyol, is from ≥ 0 milliequivalent per kg to ≤ 10 milliequivalents per kg.

10. Polyether polyol according to Claim 9, where the molar proportion of primary hydroxy groups is from ≥ 50 mol % to ≤ 100 mol %.

11. Polyether polyol according to Claim 9 with an OH number of from ≥ 10 mg KOH/g to ≤ 100 mg KOH/g.

12. Polyether polyol according to Claim 9 with an acid number of from ≥ 0.01 mg KOH/g to ≤ 5 mg KOH/g.

13. Polyether polyol composition comprising a polyether polyol according to Claim 9, and also moreover:
(A) Amines of the general formula (4): 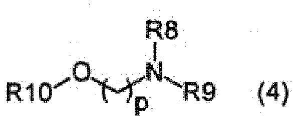 where:
R8 and R9 are mutually independently hydrogen, alkyl or aryl; or
R8 and R9 form, together with the N atom bearing them, an aliphatic, unsaturated or aromatic heterocycle;
p is an integer from 1 to 10, i.e. 1, 2, 3, 4, 5, 6, 7, 8, 9 or 10;
R10 is hydrogen, alkyl or aryl; or
R10 is -(CH₂)_{y}-N(R11)(R12), where:
R11 and R12 are mutually independently hydrogen, alkyl or aryl; or
R11 and R12 form, together with the N atom bearing them, an aliphatic, unsaturated or aromatic heterocycle;
y is an integer from 1 to 10, i.e. 1, 2, 3, 4, 5, 6, 7, 8, 9 or 10;
(B) Amines of the general formula (5): 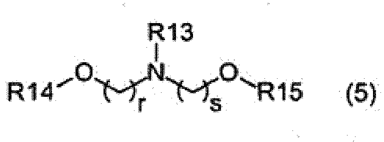 where:
R13 is hydrogen, alkyl or aryl;
R14 and R15 are mutually independently hydrogen, alkyl or aryl;
r and s are mutually independently an integer from 1 to 10, i.e. 1, 2, 3, 4, 5, 6, 7, 8, 9 or 10;
and/or:
(C) Diazabicyclo[2.2.2]octane, diazabicyclo[5.4.0]-undec-7-ene, dialkylbenzylamine, dimethylpiperazine, 2,2'-dimorpholinyldiethyl ether and/or pyridine.

14. Polyurethane polymer obtainable from the reaction of a polyisocyanate with a polyether polyol according to Claim 9 or with a polyether polyol composition according to Claim 13.

## Revendications

1. Procédé pour la préparation de polyétherpolyols présentant des groupes terminaux hydroxyle primaires, comprenant les étapes :
1. réaction d'un composé de départ présentant des atomes d'hydrogène actifs avec un époxyde de formule générale (1) : 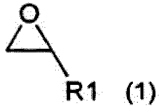 où R1 représente hydrogène, un radical alkyle ou un radical aryle et à condition que ≥ 0% en poids à ≤ 30% en poids, par rapport à la quantité totale de l'époxyde (1) utilisé, soient de l'oxyde d'éthylène,
la réaction étant réalisée en présence d'un catalyseur de type cyanure métallique double et le produit brut de cette réaction ne subissant pas d'autre purification, à l'exception d'une éventuelle étape de distillation ;
2. réaction du produit obtenu dans la 1ère étape avec un anhydride d'acide carboxylique cyclique ; et
3. réaction du produit obtenu dans la 2ème étape avec de l'oxyde d'éthylène en présence d'un catalyseur qui comprend par molécule au moins un atome d'azote, les amines tertiaires non cycliques, substituées de manière identique étant exclues, et le catalyseur utilisé dans la 3ème étape étant choisi dans le groupe comprenant :
(A) les amines de formule générale (2) : 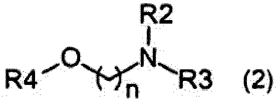 où
R2 et R3 représentent, indépendamment l'un de l'autre, hydrogène, alkyle ou aryle ; ou
R2 et R3 forment ensemble avec l'atome de N qui les porte un hétérocycle aliphatique, insaturé ou aromatique ;
n vaut un nombre entier de 1 à 10 ;
R4 représente hydrogène, alkyle ou aryle ; ou
R4 représente -(CH₂)ₓ-N(R41)(R42), où :
R41 et R42 représentent, indépendamment l'un de l'autre, hydrogène, alkyle ou aryle ; ou
R41 et R42 forment ensemble avec l'atome de N qui les porte un hétérocycle aliphatique, insaturé ou aromatique ;
x vaut un nombre entier de 1 à 10 ;
et/ou :
(B) les amines de formule générale (3) : 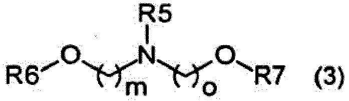 où
R5 représente hydrogène, alkyle ou aryle ;
R6 et R7 représentent, indépendamment l'un de l'autre, hydrogène, alkyle ou aryle ;
m et o valent, indépendamment l'un de l'autre, un nombre entier de 1 à 10.

2. Procédé selon la revendication 1, le composé de départ utilisé dans la 1ère étape étant un poly(oxyalkylène)-polyol présentant une fonctionnalité moyenne ≥ 2,0 à ≤ 5,0, une moyenne numérique de la masse moléculaire ≥ 62 g/mole à ≤ 1000 g/mole et un indice d'OH ≥ 100 mg de KOH/g à ≤ 1860 mg de KOH/g.

3. Procédé selon la revendication 1, où, dans l'époxyde de formule générale (1), R1 représente hydrogène, méthyle, éthyle, n-propyle, iso-propyle, n-butyle, sec-butyle, iso-butyle, tert-butyle, cyclohexyle et/ou phényle.

4. Procédé selon la revendication 1, le catalyseur de type cyanure métallique double dans la 1ère étape comprenant du zinc, du cobalt et du tert-butanol.

5. Procédé selon la revendication 1, l'anhydride de l'acide carboxylique cyclique utilisé dans la 2ème étape étant choisi dans le groupe comprenant l'anhydride de l'acide phtalique, l'anhydride de l'acide tétrahydrophtalique, l'anhydride de l'acide succinique et/ou l'anhydride de l'acide maléique.

6. Procédé selon la revendication 1, où, dans la 2ème étape, le rapport molaire d'anhydride cyclique aux groupes hydroxyle dans le produit obtenu dans la 1ère étape vaut ≥ 0,75:1 à ≤ 1,3:1.

7. Procédé selon la revendication 1, où, dans la 3ème étape, le catalyseur, qui comprend par molécule au moins un atome d'azote, se trouve, par rapport à la masse totale de la charge réactionnelle dans la 2ème et la 3ème étape, en une proportion ≥ 500 ppm à ≤ 1500 ppm.

8. Procédé selon la revendication 1, où, dans la 3ème étape, le rapport molaire d'oxyde d'éthylène aux groupes hydroxyle dans le produit obtenu dans la 1ère étape vaut ≥ 0,90:1 à ≤ 5,0:1.

9. Polyétherpolyol présentant des groupes terminaux hydroxyle primaires, pouvant être obtenus par un procédé selon la revendication 1, comprenant un bloc polyéther, un groupe hydroxyéthyle en position terminale ainsi qu'une unité diester reliant le bloc polyéther et le groupe hydroxyéthyle en position terminale et la proportion molaire de doubles liaisons terminales, par rapport à tous les groupes terminaux du polyétherpolyol, étant ≥ 0 milliéquivalent par kg à ≤ 10 milliéquivalents par kg.

10. Polyétherpolyol selon la revendication 9, la proportion molaire de groupes hydroxyle primaires valant ≥ 50 % en mole à ≤ 100 % en mole.

11. Polyétherpolyol selon la revendication 9, présentant un indice d'OH ≥ 10 mg de KOH/g à ≤ 100 mg de KOH/g.

12. Polyétherpolyol selon la revendication 9, présentant un indice d'acide ≥ 0,01 mg de KOH/g à ≤ 5 mg de KOH/g.

13. Composition de polyétherpolyol, comprenant un polyétherpolyol selon la revendication 9, ainsi qu'en outre :
(A) des amines de formule générale (4) : 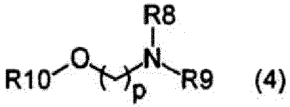 où
R8 et R9 représentent, indépendamment l'un de l'autre, hydrogène, alkyle ou aryle ; ou
R8 et R9 forment ensemble avec l'atome de N qui les porte un hétérocycle aliphatique, insaturé ou aromatique ;
p vaut un nombre entier de 1 à 10, c'est-à-dire 1, 2, 3, 4, 5, 6, 7, 8, 9 ou 10 ;
R10 représente hydrogène, alkyle ou aryle ; ou
R10 représente -(CH₂)_{y}-N(R11)(R12), où :
R11 et R12 représentent, indépendamment l'un de l'autre, hydrogène, alkyle ou aryle ; ou
R11 et R12 forment ensemble avec l'atome de N qui les porte un hétérocycle aliphatique, insaturé ou aromatique ;
y vaut un nombre entier de 1 à 10, c'est-à-dire 1, 2, 3, 4, 5, 6, 7, 8, 9 ou 10 ;
(B) des aminés de formule générale (5) : 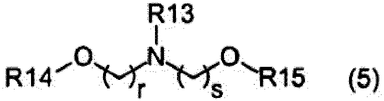 où
R13 représente hydrogène, alkyle ou aryle ;
R14 et R15 représentent, indépendamment l'un de l'autre, hydrogène, alkyle ou aryle ;
r et s représentent, indépendamment l'un de l'autre, un nombre entier de 1 à 10, c'est-à-dire 1, 2, 3, 4, 5, 6, 7, 8, 9 ou 10 ;
et/ou :
(C) du diazabicyclo[2,2,2]octane, du diazabicyclo[5,4,0]undéc-7-ène, de la dialkylbenzylamine, de la diméthylpipérazine, du 2,2'-dimorpholinyldiéthyléther et/ou de la pyridine.

14. Polymère de polyuréthane, pouvant être obtenu par la réaction d'un polyisocyanate avec un polyétherpolyol selon la revendication 9 ou une composition de polyétherpolyol selon la revendication 13.
